# EUROPÄISCHE PATENTSCHRIFT

(11) **EP 0 639 351 B1**
(45) Veröffentlichungstag und Bekanntmachung des Hinweises auf die Patenterteilung: **29.09.1999**
(21) Anmeldenummer: 94109334.6
(22) Anmeldetag: 17.06.1994
(51) Int. Cl.: A61B 17/60, A61F 2/44

(54) **Implantat zum Ersatz von Wirbelkörpern und/oder zur Stabilisierung und Fixierung der Wirbelsäule**
Implant for the replacement of a vertebra and/or stabilisation and fixation of the spinal column
Implant pour le remplacement d'une vertèbre et/ou la stabilisation de la colonne vertébrale

(30) Priorität: 20.08.1993 DE 4328062
(43) Veröffentlichungstag der Anmeldung: 22.02.1995
(73) Patentinhaber: ULRICH, Heinrich, 89075 Ulm/Donau (DE)
(72) Erfinder: Ulrich, Heinrich, D-89075 Ulm (DE); Wolf, Oleg. J.P. Dr., D-13187 Berlin (DE)
(74) Vertreter: Fay, Hermann, Dipl.-Phys. Dr.

(56) Entgegenhaltungen:
- WO-A-92/01428
- WO-A-93/01771
- FR-A- 2 636 227
- US-A- 4 892 545
- US-A- 5 192 327

## Beschreibung

Die Erfindung betrifft ein Implantat zum Ersatz von Wirbelkörpern und/oder zur Stabilisierung und Fixierung der Wirbelsäule, insbesondere bei einer Wirbelresektion, mit einem an beiden Enden Zentrierspitzen aufweisenden Stützstab zur gegenseitigen Abstützung der an das Implantat beidseits angrenzenden Wirbelkörper.

Bei aus der Praxis bekannten Implantaten dieser Art (Z. Orthop. 115 (1977) 118 - 122) besteht der Stützstab aus zwei axial miteinander fluchtenden Abschnitten, die über einen Gewindezapfen am einen Abschnitt und einem Muttergewinde am anderen Abschnitt miteinander verbunden sind, so daß durch gegenseitiges Verdrehen beider Abschnitte die Länge des Stützstabes verändert werden kann. Eine großflächige Abstützung der den Resektionsbereich begrenzenden Wirbel gegeneinander ist durch ein solches Implantat nicht möglich; daher lassen seine Möglichkeiten zur Stabilisierung und Fixierung der Wirbelsäule im Resektionsbereich zu wünschen übrig.

Der Erfindung liegt die Aufgabe zugrunde, ein Implantat der eingangs genannten Art so auszubilden, daß es auf vielseitige Weise einen Ersatz resektierter Wirbelkörper und zugleich eine zuverlässige Stabilisierung und Fixierung der Wirbelsäule im Resektionsbereich ermöglicht, sowie bei der Implantation einfach gehandhabt werden kann.

Diese Aufgabe wird nach der Erfindung dadurch gelöst, daß auf den Stützstab quer zur Stabachse ein Implantatkörper aufgeschoben und dazu mit einer seitlich an seiner Umfangsfläche offenen, den Stützstab aufnehmenden Nut versehen ist, und daß der Implantatkörper an seinen zur Anlage an den an das Implantat angrenzenden Wirbelkörpern bestimmten Stirnflächen mit einer Oberflächenstruktur versehen ist, die eine gegenseitige Fixierung der aneinanderliegenden Flächen des Implantatkörpers einerseits und der Wirbelkörper andererseits ergibt.

Der Stützstab, der in einer Reihe von passend genormten Längen zur Verfügung gehalten werden kann, läßt sich mit Hilfe seiner Zentrierspitzen sehr genau und zuverlässig an den Resektionsbereich beidseits begrenzenden Wirbelkörpern fixieren. Erst dann wird der Implantatkörper auf den Stützstab von der Seite her aufgeschoben, so daß dabei der Sitz des Stützstabes nicht mehr geändert zu werden braucht und auch nicht mehr beeinträchtigt werden kann. Der in seiner Lage durch den Stützstab ausgerichtete und fixierte Implantatkörper ergibt mit seinen Stirnflächen eine großflächige Anlage an den Wirbelkörpern, wobei der Sitz der Flächen aneinander durch die Oberflächenstruktur an den Stirnflächen des Implantatkörpers gesichert ist. Außerdem ist der Implantatkörper durch den Stützstab gegen spätere Verstellungen gehalten.

Eine besonders bevorzugte Ausführungsform des erfindungsgemäßen Implantats ist dadurch gekennzeichnet, daß der Implantatkörper quer zum Stützstab in mindestens zwei Teile geteilt ist, die mit ebenen, zur Achse des Stützstabes senkrechten Teilungsflächen aneinander liegen, daß an den Teilungsflächen je ein auf die Achse des Stützstabes zentrierter Zahnkranz vorgesehen ist und über diese Zahnkränze die aneinander liegenden Teile des Implantatkörpers in Umfangrichtung im gegenseitigen formschlüssigen Eingriff stehen, und daß die Teile des Implantatkörpers um die Achse des Stützstabes so gegeneinander verdrehbar sind, daß an jeweils unmittelbar benachbarten Teilen die Mündungen ihrer Nuten an der Umfangsfläche in Umfangsrichtung gegeneinander versetzt sind. Der dadurch erreichte Vorteil besteht zunächst darin, daß der Implantatkörper auf einfache Weise aus seinen Teilen zur jeweils benötigten Größe um den Stützstab herum aufgebaut werden kann und es somit genügt, nur eine Reihe dieser Teile in passender Abstufung ihrer Abmessungen zur Verfügung zu halten, um allen Anforderungen der Praxis durch einfache Auswahl und Zusammenstellung dieser Teile zu genügen. Außerdem sichern sich die gegeneinander verdrehten Teile gegenseitig am Stützstab gegen ein seitliches Verrutschen, insbesondere auch gegen ein Verrutschen in der durch den Nutverlauf gegebenen radialen Richtung, in der die Teile jeweils auf den Stützstab seitlich aufgeschoben worden sind.

Zweckmäßigerweise sind die Zahnkränze von aus der Teilungsfläche vorstehenden Zähnen mit radial verlaufender Zahnschneide gebildet. Entsprechend empfiehlt es sich, bei einer zur Achse des Stützstabes senkrechten und ebenen Stirnfläche des Implantatkörpers die zum Eingriff an den Wirbelkörpern vorgesehene Oberflächenstruktur durch einen Zahnkranz wie an den Teilungsflächen auszubilden. Dies bringt den Vorteil, daß Teile, die beidseits ausgebildete, also zueinander parallele und ebene Teilungsflächen aufweisen, ohne weiteres auch als im Implantatkörper endständiges Teil eingesetzt werden können, weil dann der Zahnkranz an der endständigen Stirnfläche unmittelbar die dort erforderliche Oberflächenstruktur bildet. Jedoch können auch andere der Teile auch eine zur Achse des Stützstabes geneigte Stirnfläche aufweisen, sind dann aber nur als endständiges Teil verwendbar, dessen geneigte Stirnfläche die endseitige Stirnfläche des Implantatkörpers bildet. Eine solche geneigte Stirnfläche kann aber nie eine Teilungsfläche des Implantatkörpers bilden, da die geneigte Stirnfläche ein gegenseitiges Verdrehen der beiden Teile um die Achse des Stützstabes verhindern würde. Die geneigte Stirnfläche braucht daher auch keinen Zahnkranz aufzuweisen. Vielmehr empfiehlt es sich, daß bei einer zur Achse des Stützstabes geneigten Stirnfläche die Oberflächenstruktur von aus der Stirnfläche vorstehenden Dornen gebildet ist.

Im allgemeinen wird der Stützstab einen kreiszylindrischen Querschnitt mit einem Durchmesser gleich der Breite der Nut aufweisen. Zweckmäßigerweise sind die Implantatteile als auf die Achse des Stützstabes zentrierte Kreiszylinderstücke mit zueinander parallelen oder höchstens einer zur Achse des Stützstabes geneigten Stirnfläche ausgebildet. Besitzt der Implantatkörper oder eines seiner Teile eine solche zur Achse des Stützstabes geneigte Stirnfläche, wird vorzugsweise die Anordnung so getroffen, daß die Nut sich in auf der Fallrichtung der Stirnfläche etwa senkrechter radialer Richtung von der Mitte zur Umfangsfläche hin erstreckt. Die sich bei axialer Belastung des Implantatkörpers bzw. seines Teiles durch die Flächenneigung ergebende resultierende seitlich gerichtete Kraft verläuft dann quer zur Nutrichtung, so daß sie zu keiner Verschiebung des Teils gegenüber dem Stützstab führen kann.

Der Implantatkörper oder seine Teile können axial verlaufende Löcher in Form von Durchgangs- oder Sackbohrungen aufweisen, die im implantierten Zustand ein Einwachsen von Gewebe ermöglichen. Aus gleichem Grund können der Implantatkörper bzw. seine Teile eine aufgerauhte Oberfläche besitzen, die ein Einwachsen des umgebenen Gewebes in das Implantat erleichtern. Vorzugsweise bestehen zu diesem Zweck der Implantatkörper bzw. seine Teile aus einem porösen Metallschaum.

Im folgenden wird die Erfindung an in der Zeichnung dargestellten Ausführungsbeispielen näher erläutert; es zeigen:
- Fig. 1: ein erfindungsgemäßes Implantat in einem möglichen implantierten Zustand zwischen LWK 4 und Os sacrum, in einer schematischen Seitenansicht,
- Fig. 2: eine andere Ausführungsform des erfindungsgemäßen Implantats in einer der Fig. 1 entsprechenden Darstellung,
- Fig. 3: eine nochmals andere Ausführungsform des Implantats nach der Erfindung in einer den Fig. 1 und 2 entsprechenden Darstellung zwischen LWK 5 und Os sacrum,
- Fig. 4: einen Stützstab des Implants nach den Fig. 1 bis 3 in einer Seitenansicht,
- Fig. 5: ein Teil des Implantatkörpers des Implantats nach den Fig. 1 und 2 in einer Seitenansicht,
- Fig. 6: eine axiale Draufsicht auf den Gegenstand der Fig. 5,
- Fig. 7: ein weiteres Teil des Implantatkörpers nach Fig. 1 in einer Seitenansicht und
- Fig. 8: eine axiale Draufsicht auf den Gegenstand der Fig. 7.

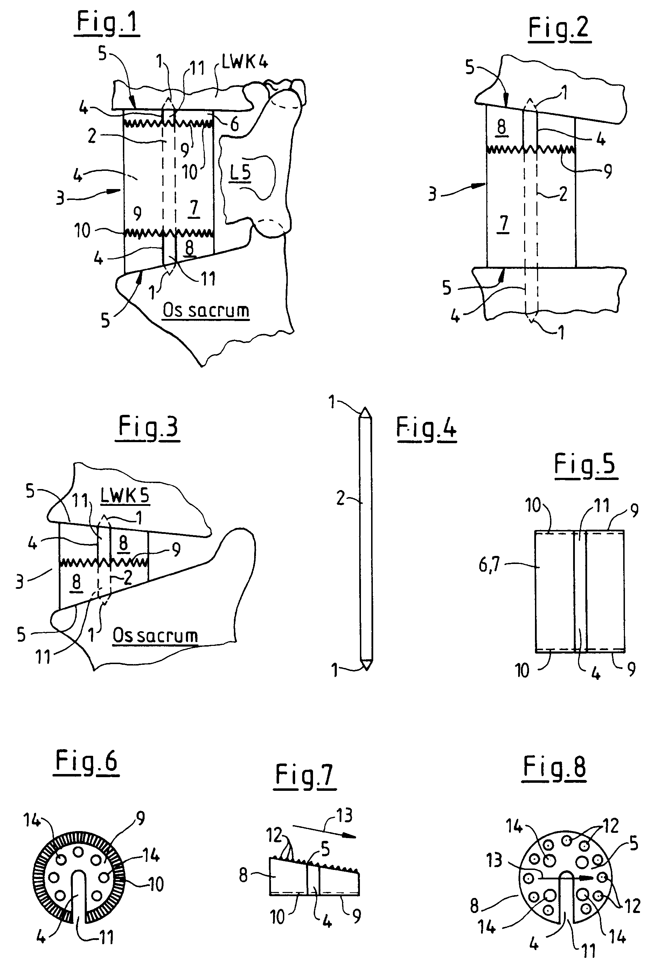

Das in den Figuren 1 und 2 dargestellte Implantat dient zum Ersatz von Wirbelkörpern und zur Stabilisierung und Fixierung der Wirbelsäule bei einer Wirbelresektion, während in Fig. 3 das Implantat lediglich die Wirbelsäule zu stabilisieren und zu fixieren hat. Dabei besteht das Implantat aus einem an beiden Enden Zentrierspitzen 1 aufweisenden Stützstab 2, der die beidseits an das Implantat angrenzenden Wirbelkörper gegeneinander abstützt. Der Stützstab 2 dringt mit seinen Zentrierspitzen 1 in die Wirbelkörper ein und ist dadurch an ihnen fixiert. Auf den Stützstab 2 ist quer zur Stabachse ein allgemein mit 3 bezeichneter Implantatkörper aufgeschoben, wozu der Implantatkörper 3 mit einer seitlich an seiner Umfangsfläche offenen, den Stützstab 2 aufnehmenden Nut 4 versehen ist. An seinen zur Anlage an den das Implantat begrenzenden Wirbelkörpern bestimmten Stirnflächen 5 ist der Implantatkörper 3 mit einer noch im einzelnen näher zu beschreibenden Oberflächenstruktur versehen, die eine gegenseitige Fixierung der aneinanderliegenden Flächen 5 des Implantatkörpers 3 einerseits und der Wirbelkörper andererseits ergibt. Der Implantatkörper 3 ist quer zum Stützstab 2 in mehrere, in Fig. 1 in drei, in den Figuren 2 und 3 in jeweils zwei Teile 6, 7, 8 geteilt. Diese Teile liegen mit ebenen, zur Achse des Stützstabes 2 senkrechten Teilungsflächen 9 aneinander. An den Teilungsflächen 9 befindet sich je ein auf die Achse des Stützstabes 2 zentrierter Zahnkranz 10. Über diese Zahnkränze 10 stehen die aneinanderliegenden Teile 6, 7, 8 des Implantatkörpers 3 in Umfangsrichtung im gegenseitigen formschlüssigen Eingriff. Dabei sind die Teile 6, 7, 8 des Implantatkörpers 3 um die Achse des Stützstabes 2 so gegeneinander verdreht, daß an jeweils unmittelbar benachbarten Teilen die Mündungen 11 ihrer Nuten 4 an der Umfangsfläche in Umfangsrichtung gegeneinander versetzt sind und sich im Ergebnis die um den Stützstab 2 herum aufgebauten Teile 6, 7, 8 gegenseitig am Stützstab verriegeln, so daß sie sich gegenüber dem Stützstab 2 nicht verschieben können. So sind in Fig. 1 die Nuten 4 der benachbarten Teile 6, 7 bzw. 7,8 um jeweils 90° gegeneinander verdreht, in Fig. 3 ebenso die Nuten 4 der Teile 7, 8; in Fig. 3 dagegen sind die Nuten 4 der beiden Teile 8 um jeweils 180° gegeneinander verdreht. In jedem Fall steht die Mittelebene der Nut 4 der Teile 8 senkrecht auf der Zeichenebene.

Die Zahnkränze 10 sind von aus der Teilungsfläche 9 vorstehenden Zähnen mit radial verlaufender Zahnschneide gebildet. Stehen an dem Teil 6, 7 beide ebenen Stirnflächen 9 senkrecht zur Achse des Stützstabes 2, so sind beide Stirnflächen mit dem Zahnkranz 10 versehen. Soweit dabei eines dieser Teile im Implantatkörper 3 endständig ist, wie das Teil 6 in Fig. 1 oder das Teil 7 in Fig 2, dient der Zahnkranz 10 zugleich als die schon früher erwähnte Oberflächenstruktur, welche die aneinanderliegenden Flächen 5 des Implantatkörpers 3 einerseits und der Wirbelkörper andererseits gegenseitig fixiert. Ist dagegen diese Stirnfläche gegenüber der Achse des Stützstabes 2 geneigt, wie dies in den Fig. 1 bis 3 das Teil 8 gilt, so ist die Oberflächenstruktur an solchen geneigten Stirnflächen 5 durch aus der Stirnfläche vorstehenden Dornen 12 gebildet. Ein Zahnkranz 10 an solchen geneigten Stirnflächen ist nicht zweckmäßig, da das Teil 8 nie mit seiner geneigten Fläche 5 an einem anderen Teil 6, 7 anliegt und also an seiner geneigten Stirnfläche keine mit dem Zahnkranz 10 benachbarter Teile 6, 7 zum Eingriff kommende Ausbildungen zu besitzen braucht.

In allen Fällen besitzt der Stützstab 2 kreiszylindrischen Querschnitt mit einem Durchmesser gleich der Breite der Nut 4. Die Stützstäbe 2 werden in Ausführungsformen abgestufter Länge zur Verfügung gehalten. Von dem sich so ergebenden Satz an Stützstäben ist in Fig. 4 nur ein einziger dargestellt. Ähnliches gilt für die Implantatteile 6, 7, 8. Auch sie stehen in einer Reihe mit abgestuften axialen Abmessungen und, bei geneigten Stirnflächen 5, abgestuften Neigungswinkeln zur Verfügung, wobei in der Zeichnung in den Fig. 5 bis 8 wiederum nur zwei Beispiele dargestellt sind. Jedoch lassen die Fig. 1 bis 3 erkennen, in wie vielseitiger Weise solche Teile zum jeweils gewünschten Implantatkörper 3 zusammengesetzt werden können.

Im einzelnen sind die Implantatteile 6, 7, 8 als auf die Achse des Stützstabes 2 zentrierte Kreiszylinderstücke ausgebildet, wobei die Kreiszylinderstücke entsprechend den Fig. 5 und 6 zwei zueinander parallele Stirnflächen 10 oder, wie in den Fig. 7 und 8 eine zur Achse des Stützstabes 2 senkrechte Stirnfläche 9 und eine geneigte Stirnfläche 5 besitzen können. Die Implantatteile 6, 7, 8 liegen jedoch im zusammengesetzten Implantatkörper 3 stets nur mit ihrer zur Achse des Stützstabes 2 senkrechten Stirnfläche 9 aneinander. Handelt es sich um ein Teil 8 mit zur Achse des Stützstabes 2 geneigter Stirnfläche 5, erstreckt sich die Nut 4 von der Mitte aus in derjenigen radialen Richtung zur Umfangsfläche hin, die auf der in den Fig. 7 und 8 durch den Pfeil 13 angedeuteten Fallrichtung der Stirnfläche 5 senkrecht steht.

Im übrigen können der Implantatkörper 3 bzw. seine Teile 6, 7, 8 axial verlaufende Löcher 14 in Form von Durchgangs- oder Sackbohrungen aufweisen, wobei sich Durchgangsbohrungen im Fall axial kurzer, Sackbohrungen im Fall axial längerer Teile empfehlen. Auch empfiehlt es sich, daß der Implantatkörper 3 bzw. seine Teile 6, 7, 8 eine aufgerauhte Oberfläche besitzen, beispielsweise aus einem Metallschaum bestehen, was dazu dient, im implantierten Zustand das Einwachsen des umgebenden Körpergewebes in das Implantat zu ermöglichen und zu erleichtern.

## Patentansprüche

1. Implantat zum Ersatz von Wirbelkörpern und/oder zur Stabilisierung und Fixierung der Wirbelsäule, insbesondere bei einer Wirbelresektion, mit einem an beiden Enden Zentrierspitzen (1) aufweisenden Stützstab (2) zur gegenseitigen Abstützung der an das Implantat beidseits angrenzenden Wirbelkörper, dadurch gekennzeichnet, daß auf den Stützstab (2) quer zur Stabachse ein Implantatkörper (3) aufgeschoben und dazu mit einer seitlich an seiner Umfangsfläche offenen, den Stützstab (2) aufnehmenden Nut (4) versehen ist, und daß der Implantatkörper (3) an seinen zur Anlage an den an das Implantat angrenzenden Wirbelkörpern bestimmten Stirnflächen (5) mit einer Oberflächenstruktur (10, 12) versehen ist, die eine gegenseitige Fixierung der aneinanderliegenden Flächen (5) des Implantatkörpers (3) einerseits und der Wirbelkörper andererseits ergibt.

2. Implantat nach Anspruch 1, dadurch gekennzeichnet, daß der Implantatkörper (3) quer zum Stützstab (2) in mindestens zwei Teile (6, 7, 8) geteilt ist, die mit ebenen, zur Achse des Stützstabes (2) senkrechten Teilungsflächen (9) aneinander liegen, daß an den Teilungsflächen (9) je ein auf die Achse des Stützstabes (2) zentrierter Zahnkranz (10) vorgesehen ist und über diese Zahnkränze (10) die aneinander liegenden Teile (6, 7, 8) des Implantatkörpers (3) in Umfangrichtung im gegenseitigen formschlüssigen Eingriff stehen, und daß die Teile (6, 7, 8) des Implantatkörpers (3) um die Achse des Stützstabes (2) so gegeneinander verdrehbar sind, daß an jeweils unmittelbar benachbarten Teilen (6, 7 bzw. 7, 8) die Mündungen (11) ihrer Nuten (4) an der Umfangsfläche in Umfangsrichtung gegeneinander versetzt sind.

3. Implantat nach Anspruch 2, dadurch gekennzeichnet, daß die Zahnkränze (10) von aus der Teilungsfläche (9) vorstehenden Zähnen mit radial verlaufender Zahnschneide gebildet sind.

4. Implantat nach Anspruch 2 oder 3, dadurch gekennzeichnet, daß bei einer zur Achse des Stützstabes (2) senkrechten und ebenen Stirnfläche (5) des Implantatkörpers (3) die Oberflächenstruktur durch einen Zahnkranz (10) wie an den Teilungsflächen (9) gebildet ist.

5. Implantat nach einem der Ansprüche 2 bis 4, dadurch gekennzeichnet, daß bei einer zur Achse des Stützstabes (2) geneigten Stirnfläche (5) die Oberflächenstruktur von aus der Stirnfläche (5) vorstehenden Dornen (12) gebildet ist.

6. Implantat nach einem der Ansprüche 1 bis 5, dadurch gekennzeichnet, daß der Stützstab (2) kreiszylindrischen Querschnitt mit einem Durchmesser gleich der Breite der Nut (4) aufweist.

7. Implantat nach einem der Ansprüche 2 bis 6, dadurch gekennzeichnet, daß die Implantatteile (6, 7, 8) auf die Achse des Stützstabes (2) zentrierte Kreiszylinderstücke mit zueinander parallelen oder höchstens einer zur Achse des Stützstabes (2) geneigten Stirnfläche (5, 9) ausgebildet sind.

8. Implantat nach Anspruch 7, dadurch gekennzeichnet, daß bei einem Implantatkörper (3) oder einem seiner Teile (6, 7, 8) mit zur Achse des Stützstabes (2) geneigter Stirnfläche (5) die Nut (4) sich in auf der Fallrichtung (13) der Stirnfläche (5) etwa senkrechter radialer Richtung von der Mitte zur Umfangsfläche erstreckt.

9. Implantat nach einem der Ansprüche 1 bis 8, dadurch gekennzeichnet, daß der Implantatkörper (3) bzw. seine Teile (6, 7, 8) axial verlaufende Löcher (10) in Form von Durchgangs- oder Sackbohrungen aufweisen.

10. Implantat nach einem der Ansprüche 1 bis 9, dadurch gekennzeichnet, daß der Implantatkörper (3) bzw. seine Teile (6, 7, 8) eine aufgerauhte Oberfläche aufweisen.

11. Implantat nach Anspruch 10, dadurch gekennzeichnet, daß der Implantatkörper (3) bzw. seine Teile (6, 7, 8) aus einem porösen Metallschaum bestehen.

## Claims

1. An implant for replacing vertebral bodies and/or for stabilising and fixing the spinal column, in particular in the event of vertebral resection, comprising a support bar (2) having centering points (1) at both ends for mutually supporting the vertebral bodies adjoining the implant on both sides, characterised in that an implant body (3) is pushed on to the support bar (2) transversely with respect to the bar axis and for that purpose is provided with a groove (4) which is laterally open at its peripheral surface and which receives the support bar (2) and that at its end faces (5) which are intended to bear against the vertebral bodies adjoining the implant the implant body (3) is provided with a surface structure (10, 12) which affords mutual fixing of the mutually contacting surfaces (5) of the implant body (3) on the one hand and the vertebral bodies on the other hand.

2. An implant according to claim 1 characterised in that the implant body (3) is divided transversely with respect to the support bar (2) into at least two parts (6, 7, 8) which bear against each other with flat division surfaces (9) which are perpendicular to the axis of the support bar (2), that provided on each of the division surfaces (9) is a respective tooth ring (10) centred on the axis of the support bar (2) and the mutually contacting parts (6, 7, 8) of the implant body (3) are in mutual positively locking engagement in the peripheral direction by way of said tooth rings (10), and that the parts (6, 7, 8) of the implant body (3) are rotatable relative to each other about the axis of the support bar (2) in such a way that at respective immediately adjacent parts (6, 7 and 7, 8 respectively) the mouth openings (11) of their grooves (4) at the peripheral surface are displaced relative to each other in the peripheral direction.

3. An implant according to claim 2 characterised in that the tooth rings (10) are formed by teeth which project out of the division surface (9) and which have a radially extending tooth cutting edge.

4. An implant according to claim 2 or claim 3 characterised in that, when the implant body (3) has a flat end face (5) which is perpendicular with respect to the axis of the support bar (2), the surface structure is formed by a tooth ring (10) as on the division surfaces (9).

5. An implant according to one of claims 2 to 4 characterised in that, in the case of an end face (5) which is inclined with respect to the axis of the support bar (2), the surface structure is formed by spikes (12) projecting from the end face (5).

6. An implant according to one of claims 1 to 5 characterised in that the support bar (2) is of a circular-cylindrical cross-section of a diameter equal to the width of the groove (4).

7. An implant according to one of claims 2 to 6 characterised in that the implant parts (6, 7, 8) are in the form of circular-cylindrical portions which are centred on the axis of the support bar (2), with mutually parallel end faces (5, 9) or at most one end face which is inclined relative to the axis of the support bar (2).

8. An implant according to claim 7 characterised in that in the case of an implant body (3) or one of its parts (6, 7, 8) having an end face (5) which is inclined with respect to the axis of the support bar (2) the groove (4) extends in a radial direction which is approximately perpendicular to the direction of fall (13) of the end face (5), from the centre to the peripheral surface.

9. An implant according to one of claims 1 to 8 characterised in that the implant body (3) or its parts (6, 7, 8) have axially extending holes (10) in the form of through bores or blind bores.

10. An implant according to one of claims 1 to 9 characterised in that the implant body (3) or its parts (6, 7, 8) have a roughened surface.

11. An implant according to claim 10 characterised in that the implant body (3) or its parts (6, 7, 8) comprise a porous metal foam.

## Revendications

1. Implant pour le remplacement de vertèbres et/ou la stabilisation et l'immobilisation de la colonne vertébrale, en particulier dans le cas d'une résection de vertèbre, comprenant une tige de support (2) pourvue de pointes de centrage (1) à ses deux extrémités pour le support mutuel des vertèbres situées de part et d'autre de la tige de support (2), caractérisé par le fait qu'un corps d'implant (3) est enfilé sur la tige de support (2), transversalement à l'axe de la tige et comporte pour cela sur le côté, dans sa surface périphérique, une rainure (4) qui reçoit la tige de support (2) et par le fait que le corps d'implant (3) est pourvu au niveau de ses surfaces frontales (5) destinées à venir en appui sur les vertèbres voisines de l'implant une structuration de surface (10, 12) qui permet une immobilisation réciproque des surfaces (5) en contact du corps d'implant (3) d'une part et des vertèbres d'autre part.

2. Implant selon revendication 1, caractérisé par le fait que le corps d'implant (3), dans la direction transversale à la tige de support (2), est divisé en au moins deux parties (6, 7, 8) qui reposent l'une sur l'autre par l'intermédiaire de surfaces de séparation (9) planes perpendiculaires à l'axe de la tige de support, par le fait qu'il est prévu sur chacune des surfaces de séparation (9), une couronne dentée (10) centrée autour de l'axe de la tige de support (2) et que les parties (6, 7, 8) en appui l'une sur l'autre du corps d'implant (3) sont en prise mutuelle par complémantarité de formes par l'intermédiaire desdites couronnes dentées (10) et par le fait que les parties (6, 7, 8) du corps d'implant (3) peuvent tourner l'une par rapport à l'autre autour de l'axe du corps d'implant (3) de telle sorte que, sur des parties directement contiguës (6, 7 ou 7, 8), les entrées (11) des rainures (4) dans la surface périphérique soient décalées l'une par rapport à l'autre dans la direction périphérique.

3. Implant selon la revendication 2, caractérisé par le fait que les couronnes dentées (10) sont formées de dents qui font saillie sur la surface de séparation (9) et dont le sommet de dent est orienté radialement.

4. Implant selon la revendication 2 ou 3, caractérisé par le fait que dans le cas d'une surface frontale (5) du corps d'implant (3) plane et perpendiculaire à l'axe de la tige de support (2), la structuration de surface est constituée d'une couronne dentée (10) comme sur les surfaces de séparation (9).

5. Implant selon une des revendications 2 à 4, caractérisé par le fait que dans le cas d'une surface frontale (5) inclinée par rapport à l'axe de la tige de support (2), la structuration de surface est formée de picots (12) formant saillie sur la surface frontale (5).

6. Implant selon une des revendications 1 à 5, caractérisé par le fait que la tige de support (2) présente une section cylindro-circulaire, avec un diamètre égal à la largeur de la rainure (4).

7. Implant selon une des revendications 2 à 6, caractérisé par le fait que dans les parties d'implant (6, 7, 8) sont conformés en tronçons de cylindre circulaire centrés autour de l'axe de la tige de support (2), avec des surfaces frontales (5, 9) mutuellement parallèles ou avec au plus une surface frontale (5, 9) inclinée par rapport à l'axe de la tige de support (2).

8. Implant selon la revendication 7, caractérisé par le fait que dans le cas d'un corps d'implant (3) ou d'une partie (6, 7, 8) de celui-ci à surface frontale (5) inclinée par rapport à l'axe de la tige de support (2), la rainure (4) s'étend dans la direction radialement sensiblement perpendiculairement à la direction de la pente (13) de la surface frontale (5), depuis le centre jusqu'à la surface périphérique.

9. Implant selon une des revendications 1 à 8, caractérisé par le fait que le corps d'implant (3) ou les parties de celui-ci (6, 7, 8) comportent des trous (10) axiaux sous la forme de trous débouchants ou de trous borgnes.

10. Implant selon une des revendications 1 à 9, caractérisé par le fait que le corps d'implant (3) ou les parties de celui-ci (6, 7, 8) présentent une surface rugueuse.

11. Implant selon la revendication 10, caractérisé par le fait que le corps d'implant (3) ou les parties de celui-ci (6, 7, 8) sont constituées de mousse métallique poreuse.
